# EUROPEAN PATENT SPECIFICATION

(11) **EP 1 144 691 B1**
(45) Date of publication and mention of the grant of the patent: **01.09.2010**
(21) Application number: 99963667.3
(22) Date of filing: 20.12.1999
(51) Int. Cl.: C12Q 1/68

(54) **PROCESS FOR THE EVALUATION AND THE MONITORING OF GENETIC VARIABILITY OF VEGETABLE SPECIES**
VERFAHREN FÜR DIE PRÜFUNG UND MONITORISIERUNG DER GENETISCHER VARIABILITÄT VON PFLANZENSORTEN
PROCEDE POUR EVALUER ET SURVEILLER LA VARIABILITE GENETIQUE D'ESPECES VEGETALES

(30) Priority: 23.12.1998 IT MI982789
(43) Date of publication of application: 17.10.2001
(73) Proprietor: CONSIGLIO NAZIONALE DELLE RICERCHE, 00185 Roma (IT)
(72) Inventor: BREVIARIO, Diego, I-20133 Milano (IT); GIANI', Silvia, I-20133 Milano (IT)
(74) Representative: Trupiano, Federica
(86) International application number: PCT/IT1999/000415
(87) International publication number: WO 2000/039334

(56) References cited:
- POWELL W ET AL: "POLYMERASE CHAIN REACTION-BASED ASSAYS FOR THE CHARACTERISATION OF PLANT GENETIC RESOURCES" ELECTROPHORESIS,DE,WEINHEIM, vol. 16, no. 9, 1 September 1995 (1995-09-01), pages 1726-1730, XP000569542
- CAETANO-ANOLLES ET AL: "Scanning of nucleic acids by in vitro amplification: New developments and applications" NATURE BIOTECHNOLOGY,US,NATURE PUBLISHING, vol. 14, no. 14, December 1996 (1996-12), pages 1668-1674-1674, XP002122510 ISSN: 1087-0156
- KOGA-BAN ET AL.: "cDNA sequences of three kinds of beta tubulins from rice" DNA RESEARCH, vol. 2, 1995, pages 21-26, XP000907121
- SUN KANG ET AL.: "Isolation and characterisation of two beta tubulin cDNA clones form rice" PLANT MOLECULAR BIOLOGY, vol. 26, 1994, pages 1975-1979, XP002137699
- VILLEMUR ET AL.: "Characterization of four new beta tubulin genes and their expression during male flower development in maize" PLANT MOLECULAR BIOLOGY, vol. 24, 1994, pages 295-315, XP002137700
- GLASS N L ET AL: "Development of primer sets designed for use with the PCR to amplify conserved genes from filamentous ascomycetes." APPLIED AND ENVIRONMENTAL MICROBIOLOGY, (1995 APR) 61 (4) 1323-30. , XP000907118
- QIN X ET AL: "Molecular cloning of three rice alpha- tubulin isotypes: differential expression in tissues and during flower development." BIOCHIMICA ET BIOPHYSICA ACTA, (1997 OCT 9) 1354 (1) 19-23. , XP000907119
- BREVIARIO D ET AL: "Three rice cDNA clones encoding different beta- tubulin isotypes." PLANT PHYSIOLOGY, (1995 JUN) 108 (2) 823-4. , XP002137701

## Description

### OBJECT OF THE INVENTION

The present invention relates to primers for the evaluation and the monitoring of genetic variability of vegetable species.

The present invention also relates to a kit for recognizing the different races of vegetable species.

### STATE OF THE ART

The development of efficient, simple and rigorous systems for the monitoring of the genetic variability that exists between the different vegetable species, for instance between cultivated plants and wild plants present within the territory, is one of the most important objects of modern Agrobiology. It is in fact desired to provide an effective instrument for the recognition and the typifying of the cultivated vegetable races that have an economic interest, in order to protect their identity, safeguarding them against possible problems of contamination and fraud and also in order to follow the development of genetic crossbreeding programs. In fact, given the extreme sophistication of the demand and offer of the agroalimentary market, increasingly rigorous controls are required to protect the marketed races. Besides, by utilizing the above systems, it possible to set up an experimental model that allows the monitoring of the cultivated and wild vegetable species existing within a given territory, in order to protect them against the danger of extinction or genetic erosion.

The problem of the conservation of biodiversity as defined internationally by the Rio de Janeiro Convention (1992) has become one of the main arguments submitted to the attention of public opinion throughout the world, as in a phase characterized by an excessive uniformity of cultures, it is necessary to preserved those genes, genotypes and genetic pools that are potentially useful in productive processes achievable through traditional methods of genetic improvement or innovating methods such as the biotechnological ones. The very norms issued by the European Union, sensitive to the protection of typical products of popular and environmental traditions, might accelerate - if suitably utilized by Regions in cooperation with Research Institutes and Farmer Associations - the achievement of the objects aiming at stopping the genetic erosion process in the more interesting areas, obtaining at the same time the valorization of niche products, with the possibility of giving spur to new processes of diversification of the species utilized. The monitoring and the determination of the genetic variability that exists between vegetable organisms are therefore objects strictly associated to the development of a competitive and far-sighted agriculture.

The problem of evaluating the genetic variability that exists between cultivated and wild plants in a reliably and rapid manner is deeply felt. Molecular genetic is at present the discipline most suitable to satisfy such need, through DNA diagnostic processes. Because of its very nature, genetic variability is intrinsically bound to polymorphisms present in the DNA molecule that however must be correctly identified and univocally associated to a given vegetable species and also to specific characteristics of agroeconomic relevance of the plant being examined.

Molecular genetic methods are rapid, ductile and reliable, and DNA diagnostic analysis has found vast application fields in the medical as well as the forensic sciences.

Molecular genetic offers at present three main solutions for the problem of monitoring genetic variability in the vegetable species, the processes applied are indicated by the following abbreviations: RFLP (Restriction Fragment Length Polymorphisms), RADP (Random Widening of Polymorphic DNA), MRP (Microsatellite Repeat Polymorphisms).

RFLP technique uses a process for the identification of DNA polymorphisms through methods of transfer of the same on nylon supports and the subsequent molecular hybridization. The technique is effective if one has a high number of molecular probes and wants to construct with them entire genetic maps. If it is adopted having the only aim of identifying genetic variability, the technique is laborious and requires the use of a high amount of genomic DNA, it cannot be automated and requires also the radioactive marking of the probes. RAPD technique and its variants (AP-PCR, DAF) are very rapid low cost techniques that need little genomic DNA and no radioactive probes. However, these techniques have a rather high risk of non-reproducibility that produces evidence of possible polymorphisms that are often, if not always, devoid of any characteristic of agronomic interest. It is substantially a mere widening of unknown and random regions of genomic DNA.

MRP technique is based on the widening of regions containing repeated DNA sequences having a high contents of specific dinucleotides (for instance, AT). Also in this case, the analysis is rapid and does not require the use of radioactive compounds or high amounts of genomic DNA. However, the identification of widened products may become laborious, as it is necessary to replace agarose gel with an acrylamide gel, and it is little informative as the sequences repeated in the genome are not always dispersed or they are located close to genes that are important for the plant. Also this technique as well as RFLP seems to be more suitable for the construction of genetic maps than for the pure and simple analysis of the existence of genetic variability.

Koga-Ban et al (DNA Resaerch, 1995, 2, 21-26) disclose a comparison among cDNA sequences deriving from three kind of rice b-tubulins. The aim of the study is to identify resistance to a specific disease of the plant in order to genetically modify similar non-resistant plants to make the resistant to the disease too.

Breviario et al. (Plant Physiology, 1995, 108 (2), 823-4) disclose the characterization of rice b-tubulin gene family by evaluation of the corresponding cDNAs.

### OBJECTS OF THE INVENTION

Object of the present invention is to provide primers of claims 1 and 2 for the evaluation and the monitoring of genetic variability of vegetable species such as to allow to establish a diversity index between the cultivated species of agronomic importance.

Another object of the invention is to provide said primers for the evaluation and the monitoring of genetic variability of vegetable species such as to allow the recognition of varietal and/or infesting contaminations and of species that are naturally resistant to pathogenic agents or environmental stresses. Another object of the invention is to provide said primers for the evaluation and the monitoring of the genetic variability of vegetable species that may be utilized for the development of new programs of genetic crossbreeding and character re-mixing.

A further object of the present invention is to provide said primers for the evaluation and the monitoring of genetic variability of vegetable species that may be associated to programs purposive to maintaining the natural biodiversity typical of a territory or an ecological niche.

Another object of the present invention is to provide said primers for the evaluation and the monitoring of genetic variability of vegetable species that is rapid, economical, precise and widely applicable.

A still further object of the invention is to provide a kit for the recognition of the genetic variability of vegetable species such as to allow to effect a rapid, reliable and economical evaluation of the race.

### DESCRIPTION OF THE INVENTION

These and still other objects and related advantages that will be better clarified by the following description are achieved by the primers and kit for the determination and the monitoring of genetic variability of vegetable species that comprises the survey of genetic polymorphisms in plant tubulines.

In particular, the primers are used in a process based on the realization of a molecular analysis such as to allow to provide evidence of the presence of genetic polymorphisms associated, in their turn, to the presence of β-tubulin isoforms that are specific for given vegetable races. Said process employs the genic family of plant β-tubulins as a natural source of genetic variability.

The process represents a new genetic molecular system that blends the ductility of RAPD system with the precision of RFLP system, is indicated by the abbreviation TBP (Tubulin Based Polymorphisms) and is based on the existence, extended to all plants, of natural polymorphic forms of β-tubulin, a protein codified by different components of a same multigenic family.

The process has proved to be rapid, economical, precise and widely applicable.

Said system is associated to the research through PCR (DNA-polymerase chain reactions) that is highly effective provided important and therefore maintained genic sequences are identified in the inside of vegetable genomes. These genic sequences must be characterizable for the presence of widely preserved zones interspersed by high variability zones. The former act as target sequences for the construction of sequences of primers that trigger the PCR reaction, while the latter act as variability source. According to the present invention, the plant β-tubulin genic family was found to be an excellent system for screening and applying the illustrated strategy. This genic family is of little numerical size and in its inside different isotypes are identifiable. The difference between the isotypes is constrained in well defined genic zones, as it cannot manifest elsewhere because of the numerous functions developed by the tubulins that are of the essential for the cell. Therefore, β-tubulin are precise genomic sequences that contain also variability polymorph elements that are identifiable. There DNA sequences are not random ones and represent molecules that are absolutely necessary for the germination, growth and development of the plant.

The characteristics of the TBP process are such as to render it applicable to all the vegetable species of agroeconomic or ecological importance, provided they contain a sufficient number, from 5 upwards, of genes codifying for β-tubulins, which requirement is largely satisfied in all the higher plants studied. The process is based on the capacity of displaying, through PCR reactions, the nucleotidic difference that exists in the hypervariable zones of the sequences codifying for β-tubulins, including also intron sequences and sequences of the promoting zones. Highly preserved sequences adjoining each of these hypervariable zones act also as triggering points for PCR reaction. The evaluation of the result and the corresponding verification of genetic diversity takes place through an analysis on agarose gel of PCR products.

The above process has allowed to obviate the main problems of the processes of the known art, i.e. complication of the processes, use of high amounts of DNA to be utilized for each individual evaluation, excessive duration of the analysis, utilization of radioactive probes, high costs to obtain the probes and start the setting up, non-reproducibility of the results, disassociation from parameters of agroeconomic importance. The process has the following advantages: it allows to realize rapid analyses, reproducible and simple and that may be extended to include all vegetable species; it is economical, requires the employment of a low amount of DNA (about 10 ng for each analysis) and does not require radioactive compounds; it allows the association of the results to parameters that are of relevance from an agrobiologic aspect, such as for instance the growth and the development of the plant.

In particular, the process has been designed and set up for the analysis of about a dozen rice cultivated races. The choice of rice as a model vegetable species is based on its being characterized by a small size genoma, the presence of a low percentage of repeated sequences, and the presence of at least 8/9 genome sequences codifying for β-tubulins that may be exploited as a variability source. Besides, from the agroeconomic aspect, rice is a wide consumption cereal, 65% of European rice production comes from Italy, and the rice production value may be estimated between 600 and 1000 billion lire/year. Control experiments carried out on both maize and rice have demonstrated the reliability of the process, as in both cases it was possible, by operating according to the experimental specifications, to widen the sequence desired and specific for that given tubulin isoform. When the experiment is carried out to allow the widening of several bands - presumable polymorphism index in the organization of rice tubulin genes - different widening pictures are obtained according to the analyzed rice race. The identity of the widened bands was verified in some cases also through the nucleotidic sequencing that confirmed that we were dealing with different sequences associated to different β-tubulin isoforms. In order to utilize the system for the identification of possible contaminants in seed lots of a given race, there were carried out also experiments of artificial blend of genomic DNA coming from two different rice races. The TBP process has allowed to identify the contamination.

Advantageously, the above TBP process is also realized employing as a material the DNA extracted from cultivated and infesting rice races, for instance cariopses rice. In fact, as said, rice is employed as a model for further studies and evaluations in the analysis system of polymorphisms, such as for instance the use of couples of new and different oligonucleotids; the use as target sequences of other hypervariability zones present in the genomic sequences of β-tubulins, and such as to act as suitable target sequences; the improvement of some technical parameters of PCR reaction. The process may be advantageously realized also with other races of rice as well as of maize and wheat, plants that are of a remarkable economic interest and whose genomic organization is very similar to rice taken, as said, as an experimental model. The above process may be advantageously employed for the determination and the monitoring of genetic variability of important cultivated plants such as horticultural and forest plants, as well as for typifying some wild, infesting and native races.

Always according to the invention, it is possible to realize a kit for the recognition of genetic diversity existing between cultivated vegetable species, based on primers subject-matter of the invention, that is utilizable on rice and cereals and other plants of agricultural interest, from the horticultural to the forest ones. For instance, the kit may be advantageously used for the monitoring of the vegetable races existing within a given territory, in order to protect the naturalistic wealth and the natural biodiversity.

As said, the kit according to the invention produces evidence of the diversity existing between vegetable races of the same plant, exploiting the molecular heterogeneity that is present in the genes that codify for β-tubulin, a protein that is of the essential for the life, the growth and the development of the plant. The main characteristics that are exploited are in number of two. In each plant, β-tubulin is codified for a certain number of genes and not one only gene, which is a first variability source. In each gene that codifies for β-tubulins, preserved nucleotidic sequences and variable sequences are recognizable and the latter are the second variability source. To sum up, the kit according to the present invention is composed by a variability source represented by the genomic DNA extracted from the plant, two oligonucleotidic sequences (primers) that are able to associate to β-tubulin gene sequences close to the variability zones, an enzymatic reaction that is able to widen the DNA sequence contained between the primers, an agarose gel through which it is possible to point out the widened DNA bands, possibly different for the different analyzed races.

Some examples of practical realization are reported in the following by way of non limiting example of the present invention.

### Reagents of the TBP kit

### Plant genomic DNA:

Genomic DNA may be extracted from any tissue of the plant. The results we report are based on DNA extraction from rice coleoptiles.

The extraction protocol includes cold pulverization (in the presence of liquid nitrogen) of 1 g coleoptides and the subsequent transfer of the powder into a tube containing 6 ml of an extraction buffer composed as follows: 48% urea, 0,35M sodium chloride, 0,05M Tris-HCl pH 7,5, 0,02M EDTA pH8, 2% Sarkosyl, 100 mg/ml RNAse. Upon dissolution of the powder in the buffer, DNA is extracted with a phenol/chloroform mixture (25 parts phenol/24 parts chloroform/1 part isoamylic alcohol), previously adjusted to a pH 8 with Tris-HCl. The extraction is followed by 3000r centrifugation for 10 min, which allows the separation of the DNA-containing aqueous phase from the organic one. The aqueous phase is transferred into a small becker and DNA is percolation-precipitated with cold absolute ethyl alcohol. DNA is literally collected on the ends of glass sticks in the same way as a small wool ball (spooling technique).

Having been air-dried for a short time, DNA is resuspended in bidistilled water and its concentration is brought to 1 mg/ul. These DNA stocks, obtained from each race, are then preserved at -20°C until their utilization.

### Triggering primers

In the kit developed till now, triggering is caused to coincide with nucletodic sequences proximal to codon 132-1 of the genes codifying for β-tubulin, recognizing in that point the emergence of a possible variability, associated to the constant presence in all the plants analyzed till now of an intron or non-codifying sequence. At present, two types of couple of primers have been developed.

The first couple is composed by two sequences, one upstream (Tub16sp. /fw) and one downstream (Tubl6sp./rev) of codon 132-1 of a specific gene of the rice β-tubulin family (TubB16 isotype, cloned by us). This couple of primers allows the widening of the hypervariable nucleotidic sequence specific for the TubB16 gene.

The primer sequences are:
TubB16sp./fw: 5'-TCTGGTGCTGGTAACTGG-3'
TubB16sp./rev: 5'-GAGAATGTCAGCATCATGCG-3'

The second couple is composed by sequences that are always upstream (BTKd1-fw) and downstream (BTKd1-rev) of codon 132-1, but able, by virtue of small base change, to cause the emergence of a variability picture that is no longer specific for the TubB16 gene only.

In this case the sequences are:
BTKd1-fw : 5'AGGCTGAGAACTGCGACTGC-3'
BTKd1-rev: 5'GAGAATGTGAGCATCATGCG-3'

All the sequences of primers are preserved as stock solutions at -20°C and at the concentration of 2 mg/ul.

### Reagents necessary for PCR widening reaction of the zones of nucleotidic variability

Mixture of deoxynucleotides (dATP, dCTP, dGTP, TTP) employed at the final concentration, for each deoxynucleotide, of 187,5 mM for each analysis assay. Magnesium chloride (MgCl₂): employed at the final concentration of 1,5 mM for each analysis assay.

Taq polymerase: 0,25 enzyme units for each analysis assay in a reaction buffer containing 50 mM potassium chloride (KCl) , 10 mM Tris-HCl pH 9 and 0,1% Triton X-100.

### Widening protocols

Two examples are reported, one that utilizes more strict conditions (PCR1-BTKd), and the other one that utilizes more moderate conditions (PCR2-BTKd).

The PCR1-BTKd widening protocol includes the following steps:
1. 95°C for 3 min, 1 cycle
2. 95°C for 20 sec
3. 58°C for 20 sec
4. 72°C for 2 min
   Steps 2-4 are repeated for 5 cycles
5. 95°C for 20 sec
6. 56°C for 20 sec
7. 72°C for 2 min
   Steps 5-7 are repeated for 5 cycles
8. 95°C for 20 sec
9. 54°C for 20 sec
10. 72°C for 2 min
   Steps 8-10 are repeated for 20 cycles
11. 72°C for 20 min, 1 cycle
12. 60°C for 10 min, 1 cycle
13. 15°C until the collection of the tubes and the analysis of the products

The PCR2-BTKd widening protocol includes the following steps:
1. 6 min to reach 91°C
2. 95°C for 3 min, 1 cycle
3. 95°C for 20 sec
4. 54°C for 20 sec
5. 72°C for 2 min
   Steps 3-5 are repeated for 30 cycles
6. 72°C for 20 min, 1 cycle
7. 60°C for 10 min, 1 cycle
8. 15°C until the collection of the tubes and the analysis of the products.

### Analysis of widening products

The visualization of widened DNA bands is made by separating them on plates of agarose gel, at a concentration of the latter from 1,4% to 1,8%, according to the experimental requirements. The DNA is visualized by ethidium bromine stain used at the final concentration of 100 ng/ml.

### EXPERIMENTAL RESULTS

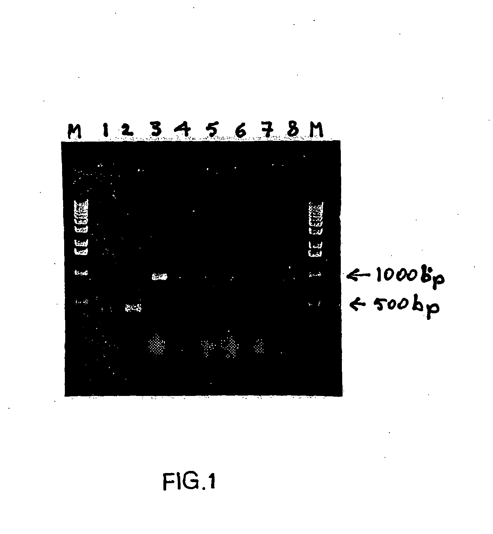

1. Figure 1 shows the widening of a band of about 900 base couples specific for the β-tubulin rice gene TubB16.

This experiment was carried out to demonstrate the feasibility of the experimental approach when one takes also into consideration the widening of one only sequence of one only β-tubulin gene. The figure shows that this is possible both in maize (positions 1 and 2) and rice (positions 3-8).

The band of 419 base couples obtained from the analysis of 100 ng or 1 mg of maize genomic gene is the one expected for an intron in the position 132-1 of 89 base couples (already described in the literature). The analysis on maize has been obviously carried out with primers that are specific for that plant and for a specific β-tubulin gene (ZmTubB4). The band of about 900 base couples present from position 3 to 8 represents instead the intronic sequence of TubB16 gene in 6 rice races that was not previously known. The band sequencing data we could obtain demonstrate irrefutably that it was the intron of rice TubB16 gene.

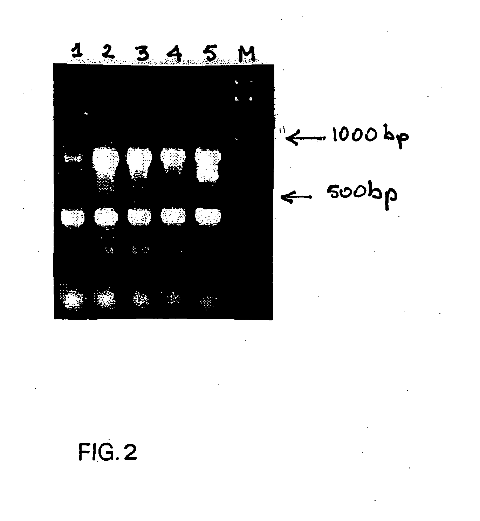

As concerns rice, the analysis was carried out using 1 mg of genomic DNA, the specific couple of primers (Tub16sp. /fw and Tub16sp. /rev) and the widening protocol (PCR1-BTKd). The rice races analyzed in this experiment are respectively, starting from position 3: Arborio, 4 Roma, 5 Ariete, 6 IR72, 7 Thaibonnet, 8 Elio, M = reference marker for the molecular size. 2. Figure 2 shows the widening of several bands that constitute a specific molecular picture for five different rice races.

This experiment was carried out at a concentration of the genomic DNA of 1 mg, BTKd1-fw and BTKd1-rev couple of primers, PCR1-BTKd widening protocol. The rice races analyzed in this experiment are respectively:
1. M2O2, 2. IR72, 3. Arborio, 4. Ariete, 5. Roma, M = reference marker for the molecular size.

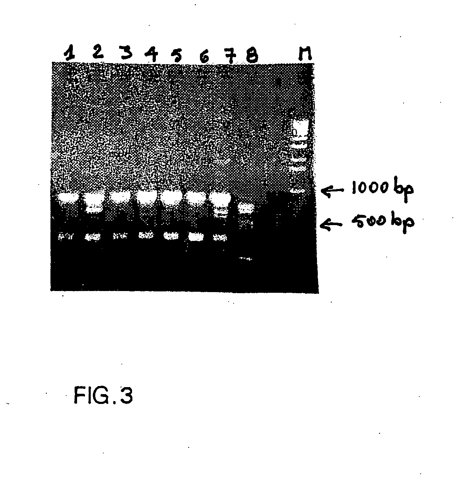

3. Figure 3 shows the widening of several bands that constitute a specific molecular picture for seven different rice races. The figure also show a sample of maize DNA (position 8).

This experiment was carried out reducing the amount of the starting genomic DNA from 1 mg to 100 ng. BTKd1.fw and BTKd1. rev. couple of primers, PCR1-BTKd widening protocol. The rice races analyzed in this experiment are respectively:
1. Arborio, 2. Roma, 3. Ariete, 4. IR72, 5. Thaibonnet, 6. M203, 7. Elio, M = reference marker for the molecular size.
4. Widening of several bands that constitute a specific molecular picture for seven different rice races.

Figure 4 shows the same rice races of Figure 3 which have been analyzed with the PCR2-BTKd widening protocol.

The rice races analyzed in this experiement are respectively:
1. Arborio, 2. Ariete, 3. Roma, 4. IR72, 5. Elio, 6. Thaibonnet, 7. M203, M = reference marker for the molecular size.

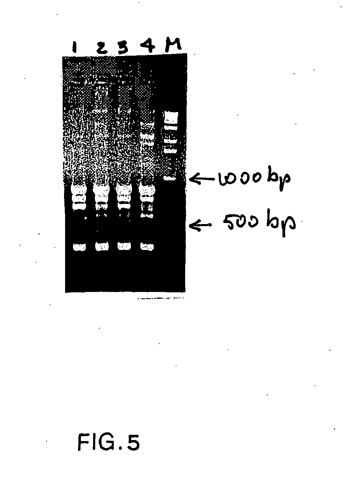

Figure 5 shows the identification test through the use of the TBP contamination kit between two different rice races (Elio and Roma).

The experiment was prepared by mixing different amounts of genomic DNA obtained from the two cultivated races Roma and Elio. The widening protocol used was PCR2-BTKd with BTKd1-fw and BTKd1-rev couple of primers
pos. 1 100 ng Roma
pos. 2 50 ng Roma + 100 ng Elio
pos. 3 20 ng Roma + 100 ng Elio
pos. 4 100 ng Elio
M = reference marker for the molecular size.

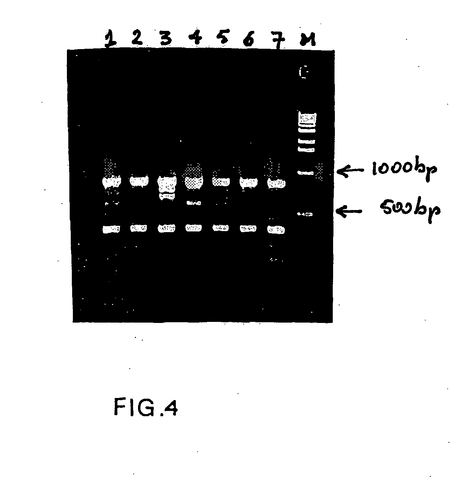

The TBP kit according to the present invention as developed allows to produce evidence of a specific picture for cultivated rice races associated to the presence of distinctive bands of widened DNA (Fig. 4). As has been evidenced, the diversity picture is more or less evident according to the experimental conditions utilized. This leaves open the possibility of further studies associated to the introduction and the modification of parameters such as:
- identification of target sequences known as being variable in the gene of plant β-tubulin,
- identification of couples of primers such as to produce evidence of other or similar diversities,
- setting up of modified conditions of the widening reaction associated to different temperatures, cycles, enzyme amount and saline concentrations.

The TBP process is utilized for recognizing different rice races but may be also applied to many vegetable species, provided they contain the fundamental base of variability pointed out by this kit, i.e. several genes that codify for β-tubulin.

## Claims

1. Oligonucleotidic sequences 5'-TCTGGTGCTGGTAACTGG-3' (TubB16sp./fw) and 5'-GAGAATGTCAGCATCATGCG-3' (TubB16sp./rev) that may be associated to the genic sequence of plant b-tubulin.

2. Oligonucleotidic sequences 5'-AGGCTGAGAACTGCGACTGC-3' (BTKdl-fw) and 5'-GAGAATGTGAGCATCATGCG-3' (BTKdl-rev) that may be associated to the genic sequence of plant b-tubulin.

3. A kit for the recognition of the different races of vegetable species which comprises one or more oligonucleotidic sequences according to claims 1 or 2.

4. Use of the kit of claim 3 for the determination of the different races.

## Patentansprüche

1. Oligonukleotidsequenzen 5'-TGTGGTGGTGGTAACTGG-3' (TubB16sp./fw) und 5'-GAGAATGTGAGCATGATGCG-3'(TubB16sp./rev), die an die Gensequenz von pflanzlichem β-Tubulin binden können.

2. Oligonukleotidsequenzen 5'-AGGCTGAGAACTGCGACTGC-3'(BTKdl-fw) und 5'-GAGAATGTGAGCATCATGCG-3' (BTKdl-rev), die an die Gensequenz von pflanzlichem β-Tubulin binden können.

3. Ein Kit zur Erkennung von unterschiedlichen Sorten von Pflanzenarten enthaltend eine oder mehrere Oligonukleotidsequenzen gemäß Anspruch 1 oder 2.

4. Verwendung des Kits gemäß Anspruch 3 zur Bestimmung von unterschiedlichen Sorten.

## Revendications

1. Séquences d'oligonucléotides 5.'-TCTGGTGCTGGTAACTGG-3' (TubB16sp./fw) et 5'-GAGAATGTCAGCATCATGCG-3' (TubB16sp./rev) qui peuvent être associées à la séquence génétique de la b-tubuline de plantes.

2. Séquences d'oligonucléotides 5'-AGGCTGAACTGCGACTGC-3' (BTKdl-fw) et 5'-GAGAATGTGAGCATCATGCG-3' (BTKdl-rev) qui peuvent être associées à la séquence génétique de la b-tubuline de plantes.

3. Kit de reconnaissance de différentes variétés d'espèces végétales qui comprend une ou plusieurs séquences d'oligonucléotides selon les revendications 1 ou 2.

4. Utilisation d'un kit selon la revendication 3 pour l'identification de différentes variétés.
